(19) Europäisches Patentamt
European Patent Office
Office européen des brevets

(11) Publication number: 0 104 717
A1

# (12) EUROPEAN PATENT APPLICATION

(21) Application number: 83303684.1

(22) Date of filing: 27.06.83

(51) Int. Cl.³: C 07 D 213/74

(30) Priority: 23.09.82 US 422411

(43) Date of publication of application: 04.04.84 Bulletin 84/14

(84) Designated Contracting States: AT BE DE FR GB IT NL SE

(71) Applicant: CHEMTRONICS, INC.
Old Bee Tree Road
Swannanoa North Carolina 28778(US)

(72) Inventor: Hudson, Frederick Mitchell
9 Valle Vista Drive
Asheville North Carolina 28804(US)

(74) Representative: Wain, Christopher Paul et al,
Northumberland House 303-306 High Holborn
London WC1V 7LE(GB)

## (54) Method of preparing 2,6-bis(picrylamino)-3,5-dinitropyridine.

(57) The explosive, 2,6-bis(picrylamino)-3,5-dinitropyridine is prepared by
(a) reacting, in a polar protic solvent, 2,6 diaminopyridine with at least one compound having the general formula

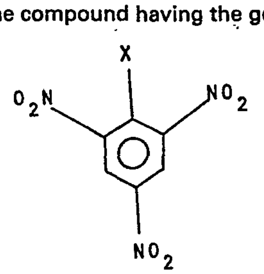

wherein:
X is a halogen or an alkoxy group containing 1 to 4 carbons, provided however that when X is a halogen, at least one base material selected from alkaline earth metal carbonates and alkali metal bicarbonates, is present in an amount of at least one molar equivalent to said compound,
(b) filtering the reaction product of step (a); and
(c) nitrating the filtered reaction product of step (b) with at least 90 percent nitric acid at a temperature in the range of about 32°F (0°C) to about 85°F (30°C).

EP 0 104 717 A1

# METHOD OF PREPARING 2,6-BIS(PICRYLAMINO)-3,5-DINITROPYRIDINE

This invention relates to an improved method of making the thermally stable high explosive 2,6-bis(picrylamino)-3,5-dinitropyridine.

United States Patent no. 3,678,061 describes the preparation of 2,6-bis(picrylamino)-3,5-dinitropyridine, hereinafter for convenience referred to as PANPX, by a two-step synthesis in which, in the first step, 2 moles of picryl chloride are reacted with 1 mole of 2,6-diaminopyridine to form the intermediate compound 2,6-bis-(picrylamino)pyridine, hereinafter for convenience referred to as BPAP, and then, in the second step, the intermediate BPAP is reacted with 90 percent nitric acid to form the desired product PANPX. The U.S. patent specifically requires that the preparation of the intermediate BPAP be conducted in the presence of a polar, aprotic solvent, such as N,N-dimethylformamide, and in addition, strongly urges that sodium fluoride, in at least molar equivalent quantity to the picryl chloride, be added to the intermediate reaction mixture in order to double the yield of the BPAP. It is explained that the sodium fluoride, in the presence of the polar, aprotic solvent, converts the picryl chloride to picryl fluoride which is more reactive with 2,6-diaminopyridine than is picryl chloride, in that picryl fluoride is more receptive than picryl chloride to nucleophilic substitution.

During the course of the preparation of the intermediate BPAP by this method, there is generated both hydrofluoric acid and hydrochloric acid. It is believed that the hydrochloric acid is a by-product of the reaction between picryl chloride and 2,6-diaminopyridine, and that the hydrofluoric acid is a by-product of the reaction between picryl fluoride and 2,6-diaminopyridine. It is well known that hydrofluoric acid is very corrosive of glass and/or glass lined reaction vessels, and that hydrochloric acid is very corrosive of stainless steel equipment.

In addition to the formation of the undesirable by-product acids, the above process has the disadvantage that, in order to proceed to completion, it must be conducted under rigorous conditions, i.e. 115°C for 5 hours. Furthermore, the intermediate BPAP, being highly soluble in the aprotic solvent, must be recovered by pouring the entire intermediate reaction mixture into water which promotes precipitation of very finely divided particles of the intermediate. The finely divided particles of intermediate BPAP are very difficult to filter and wash free of impurities such as residual quantities of sodium fluoride and sodium chloride which, if present in the second step -- the nitration step -- result in the (undesirable) formation of hydrofluoric acid and hydrochloric acid. Accordingly, to avoid, or at least reduce, the formation of undesirable acid species in the nitration step, the intermediate product must be purified by extensive recrystallization procedures prior to commencement of the second step.

We have now devised an improved method of preparing PANPX which avoids or reduces the problems referred to above.

According to the present invention, there is provided a method of preparing 2,6-bis(picrylamino)-3,5 dinitropyridine, comprising:

(a) reacting, in a polar protic solvent, 2,6-diaminopyridine with at least one compound having the general formula

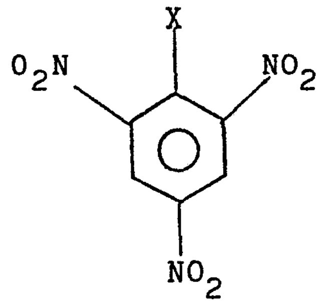

wherein:

X is a halogen or an alkoxy group containing 1 to 4 carbons provided however that when X is a halogen, at least one base material selected from alkaline earth metal carbonates and alkali metal carbonates, is present in an amount of at least one molar equivalent to said compound;

(b) filtering the reaction product of step (a); and,

(c) nitrating the filtered reaction product of step (b) with at least 90 percent nitric acid at a temperature in the range of about 32°F (0°C) to about 85°F (30°C).

In the method of this invention, the formation of by-product acid is eliminated or at least substantially reduced, and in a preferred method the formation of hydrofluoric acid is eliminated and the formation of hydrochloric acid is substantially reduced if not completely eliminated. Furthermore, by the method of this invention, the intermediate product BPAP, being substantially insoluble in the reaction solvent, is readily isolated in yields comparable to those provided by the preferred method of U.S. 3678061 and, in addition, extensive purification of the intermediate is not required prior to performance of the nitration step.

In the method of the invention, the intermediate

product 2,6-bis(picrylamino)pyridine is readily prepared by the reaction of 2,6-diaminopyridine with a trinitrobenzene derivative selected from trinitrobenzene alkyl ethers and trinitrobenzene halides in the presence of a polar, protic solvent, such as aqueous alcohol. The reaction proceeds as rapidly as the reactant materials are combined at a temperature corresponding to the atmospheric reflux temperature of the alcohol component of the protic solvent. The intermediate product is crystallized in a highly pure state as it forms in the solvent, which enables rapid separation of the solid intermediate from the reaction liquor without need for recrystallization prior to nitration. When the trinitrobenzene derivative reactant is a trinitrobenzene halide, the by-product acid referred to above, which must be eliminated from the reaction, is removed by reacting it with a basic material such as an alkaline earth metal carbonate or an alkali metal bicarbonate. The preferred trinitrobenzene halide is picryl chloride and the preferred base is magnesium carbonate. However, when the trinitrobenzene derivative reactant is a trinitrobenzene alkyl ether, the by-product acid referred to above is not produced at all and therefore the base material is not required.

It has also been discovered that nitration of the intermediate product of the method of this invention can produce the desired product, PANPX, having a higher thermal stability than that produced according to the method of the U.S. patent referred to above.

During the second step -- the nitration step -- of the method of this invention, the intermediate product BPAP is added to at least 90 percent nitric acid, and more preferably 98 percent nitric acid, at a rate sufficient to maintain the temperature of reaction in the range of from about 32°F (0°C) to about 85°F (30°C). After addition of the BPAP, the temperature is increased to about the atmospheric reflux temperature of the nitric acid and then,

after reaction has proceeded for about 5 to 7 hours, approximately 20 to 35 percent by weight of the original nitric acid charge can be distilled from the reaction mixture. Upon this removal of the nitric acid, the temperature of reaction has been observed to increase to a range of from about 185°F (85°C) to about 200°F (83°C). At this time, the reaction mass is allowed to cool to ambient and lower.

The product PANPX produced by the method of this invention has an unexpectedly higher thermal stability than the product produced by the U.S. patent. As determined by differential scanning calorimetry, the product according to the U.S. patent decomposes at 350°C; however, the product produced by the method of this invention utilizing 98% nitric acid is stable to 370°C when nitric acid is not distilled from the system, and it is stable to 379°C when nitric acid is removed from the system as previously described.

It is believed that the following theory may explain the reactions involved in the production of the intermediate product BPAP, but it is to be expressly understood that the results obtains herein shall not be unduly limited by this proposed theory, whether or not it be correct. The intermediate reaction equation is set out below:

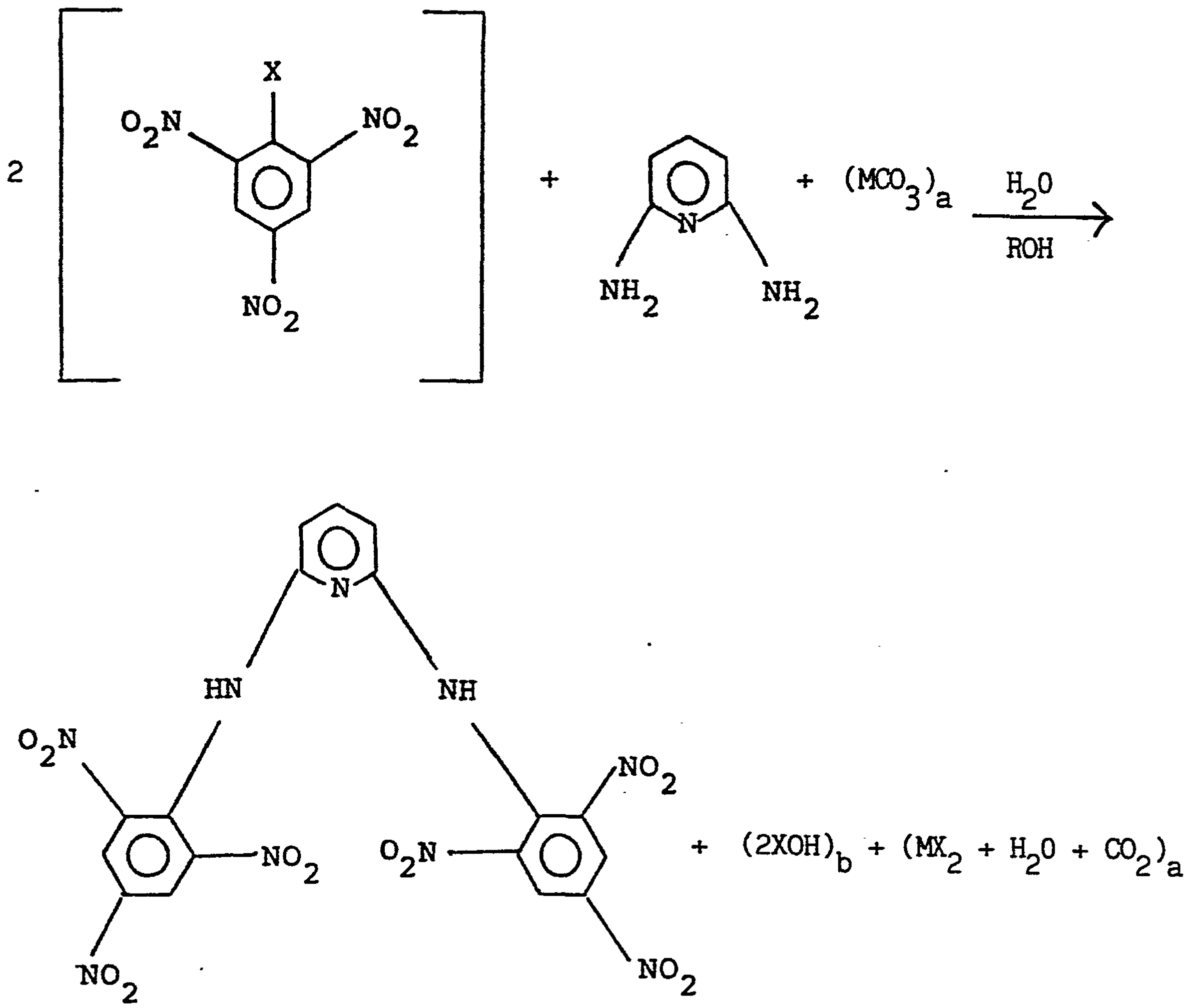


wherein:

X is a halogen or an alkoxy group, preferably F, Cl, I, Br and $OR_1$, more preferably Cl; when X is a halogen "a" is 1 and "b" is zero, and when X is an alkoxy group "a" is zero and "b" is 1;

M is an alkaline earth or an alkali metal, preferably Mg, Ca, Ba, Sr, Na, K, and Li; more preferably Mg. When M is Na or K the basic material ($MCO_3$) is a bicarbonate; and

R and $R_1$ are selected from saturated aliphatic radicals having 1 to 4 carbons, preferably methyl, ethyl, n-propyl, isopropyl, tert-butyl and, more preferably, R is isopropyl and $R_1$ is methyl.

When "b" is 1 and "a" is zero, the undesirable hydrogen halide is not formed at all; therefore, it need not be removed as described below. When "a" is 1, there is irreversible formation of the metal halide, $MX_2$, and of the $CO_2$, which is permanently removed from the reaction mixture as a gas. In the reaction the base material -- the metal carbonate, or bicarbonate, $MCO_3$ -- reacts with hydrogen halide, e.g. HCl or HF, as it is formed to thus remove from the reaction mixture the objectionable by-product acid referred to above.

It is believed that the elimination or removal of the hydrogen halide, e.g. HCl, promotes the desired reaction between the trinitrobenzene derivative and the diaminopyridine, otherwise the relatively unreactive, positively charged, diamino-pyridinium ion

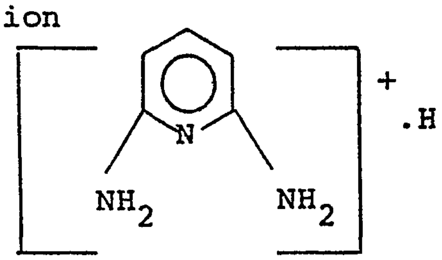

is formed by reaction of the diaminopyridine and hydrogen halide which effectively removes diaminopyridine from the desired reaction in the absence of the extreme conditions, 115°C, 5 hours, specified in the U.S. patent.

The base material utilized is thus critical to the complete reaction between the trinitrobenzene halide and the 2,6-diaminopyridine. The function of the base, as described above, is to permanently remove the hydrogen halide from the reaction mixture in order to prevent the formation of the diamino-pyridinium ion, but at the same time the base must not be a sufficiently effective nucleophile to itself react with the trinitrobenzene derivative, nor active enough to generate a competitive coordinate base from the reaction solvent. Accordingly, the base selected must be active enough to react with materials which would otherwise undesirably react with the diaminopyridine, but not active enough to effectively compete with the diaminopyridine for the trinitrobenzene derivative. In other words, the base material utilized must have greater basic strength, but less nucleophilicity than diaminopyridine. It is believed that the carbonate and bicarbonate materials specified above satisfy the above stringent requirements.

The quantity of base utilized is at least a molar equivalent to the trinitrobenzene derivative.

The selection of protic solvents is also an important feature of this invention. In this connection, water serves to solubilize the 2,6-diaminopyridine for reaction and also serves as an ionization medium for the formed acids and added base materials in order to promote that essential reaction which was referred to above. Also, the second protic solvent the alcohol, serves to solubilize the trinitrobenzene derivative for reaction and to promote crystallization of the intermediate BPAP as it is formed. Generally, the volume of water utilized is from about 10 percent by volume to about 50 percent by volume of the alcohol. Generally, the quantity of alcohol utilized is from about 5 to about 15 times by weight of the trinitrobenzene derivative.

In order that the invention may be more fully understood, embodiments thereof will now be described with reference to the accompanying schematic drawing of an apparatus for carrying out the method.

Referring to the drawing, a quantity of water is first introduced into vessel 2 equipped with stirrer 3 via line 4 and mixed therein with a quantity of 2,6-diaminopyridine, which is introduced into vessel 2 via line 6. At substantially the same time as the above is taking place, a quantity of alcohol, preferably isopropyl alcohol, is first introduced into vessel 8 equipped with stirrer 7 via line 10 and then a quantity of one or more trinitrobenzene derivatives, preferably a trinitrobenzene halide and more preferably picryl chloride, is introduced into vessel 8 via line 12 and a quantity of one or more base materials, preferably an alkaline earth carbonate, and still more preferably magnesium carbonate, is introduced into vessel 8 via line 14. The materials introduced into vessels 2 and 8, respectively, are permitted to remain in the vessels for a time sufficient for complete dissolution of the organic materials to occur. The contents of each of vessels 2 and 8 can be stirred and heated in order to permit rapid dissolution.

With respect to the quantity of trinitrobenzene derivative introduced into vessel 8 relative to the quantity of

2,6-diaminopyridine introduced into vessel 2, there should be at least 2 moles and preferably 2.1 to 2.5 moles of trinitrobenzene derivative per mole of 2,6-diaminopyridine. In addition, the quantity of base introduced into vessel 8 via line 14 should be the molar equivalent of the quantity of trinitrobenzene derivative introduced via line 12. It should be noted that if the trinitrobenzene derivative introduced via line 12 is a trinitrobenzene halide, then a base is introduced via line 14. However, if the trinitrobenzene derivative is not a trinitrobenzene halide, but is instead a trinitrobenzene alkyl ether, then no base is required and nothing is introduced into vessel 8 via line 14.

When the contents of vessel 2 and 8 have been dissolved, all of the contents of vessel 8 are then first introduced into reactor vessel 16 equipped with stirrer 17 via line 18. After all contents of vessel 8 have been introduced into reactor vessel 16, the contents of vessel 2 are then slowly introduced into reactor vessel 16 via line 20 at a rate effective to control the evolution of $CO_2$, which is a by-product of the reaction which occurs in vessel 16 between the trinitrobenzene halide and the 2,6-diaminopyridine. When the contents of vessel 8 are introduced into reactor 16, the contents of the reactor 16 are heated to the atmospheric reflux temperature of the alcohol solvent. When the alcohol is isopropyl alcohol, the reflux temperature is approximately 82°C. The alcohol in gas phase is vented from reactor vessel 16 and introduced via line 22

into reflux condenser 24 wherein the alcohol is condensed and returned in liquid phase to vessel 16 via line 26. Carbon dioxide, formed in reactor vessel 16 is also vented from vessel 16 and introduced via line 22 into condenser 24 and vented from condenser 24 and introduced via line 28 into a lower portion of scrubber 30 wherein it is contacted by a descending stream of sodium hydroxide which is introduced into an upper portion of scrubber 30 via line 32. The scrubbed material is removed from scrubber 30 and introduced into receiver 36 via line 34 for recirculation to scrubber 30 via lines 37, 32 and pump 38. Scrubber 30 is vented to the atmosphere via line 40.

The reaction in reactor 16 between the trinitrobenzene derivative and the 2,6-diaminopyridine is complete upon the addition of all the contents of vessel 2 into reactor 16. Accordingly, no residence time is required in reactor 16 once all the contents of vessels 8 and 2 have been introduced into reactor 16.

The reaction mass in reactor 16, which is a slurry of solid in liquid, is removed from reactor 16 and introduced via line 42 into vacuum separator 44. In separator 44 the slurry introduced via line 42 is first separated into a filtrate and a solid. The filtrate is removed from separator 44 via line 46. The filtrate removed via line 46 is comprised of alcohol, water, magnesium salts, and excess trinitrobenzene derivative with traces of other raw materials and by-products. After this initial separation, warm water is introduced

into vacuum separator 44 via line 48 in a quantity sufficient to dissolve any remaining water soluble material from the solid product contained in vacuum separator 44. The solid material in separator 44 is the intermediate product of the invention, 2,6-bis(picrylamino) pyridine, which has been referred to hereinabove as BPAP.

The BPAP intermediate can be removed from separator 44 via line 50 as a damp solid and introduced into dryer 52, wherein dryer 52 the damp solid is subjected to a temperature of approximately 100°C and maintained in dryer 52 until the quantity of volatile material remaining in the intermediate is less than or equal to 0.5 percent by weight of the dried BPAP product. The thus dried BPAP can then be removed from dryer 52 via line 54 for storage or it can be utilized in nitration reactor 56 as hereinafter described. The dried BPAP can be introduced into nitration reactor 56 via line 58. During the drying process in dryer 52, the volatile materials are removed via line 60 and vented to the atmosphere. The intermediate product contained in line 54 is a bright yellow/orange crystalline material which is free-flowing and somewhat powdery. The yield of intermediate BPAP is approximately 90 percent of theoretical based upon the quantity of trinitrobenzene derivative introduced into vessel 8 via line 12 and 2,6-diaminopyridine introduced into vessel 2 via line 6.

In a preferred method of operation, the damp intermediate is removed from separator 44 and introduced via line 62 into nitration reactor 56. However, prior to the intro-

duction of the intermediate into reactor 56, 98 percent nitric acid is introduced into reactor 56 via line 64. The intermediate product BPAP is introduced into reactor 56, after the entire charge of nitric acid is introduced into the reactor, preferably through line 62 as a damp solid, and alternately via line 58 as a dry solid. The intermediate is introduced at a rate sufficient to maintain the reaction temperature in reactor 56 at a value between 32°F and 80°F.

When all of the intermediate product has been introduced into reactor 56, the contents of the reactor are heated slowly to the atmospheric reflux temperature of the nitric acid, which temperature is approximately 170°F. Meanwhile, the contents of reactor 56 are stirred with stirring apparatus 66. Nitric acid in the vapor phase is vented from nitration reactor 56 and introduced via line 68 into reflux condenser 70 and condensed and returned in the liquid phase to reactor 56 via lines 72 and 74. Also removed from reactor 56 via line 68 are non-condensable materials such as nitrogen oxide gases. These are introduced into condenser 70 and removed from condenser 70 and introduced via line 76 into a lower portion of scrubber 78 wherein they are contacted by a descending stream of sodium hydroxide which is introduced into an upper portion of scrubber 78 via line 80. Excess sodium hydroxide in scrubber 78 and scrubbed material is removed from scrubber 78 and then introduced via line 82 into receiver 84 for recirculation to scrubber 78 via pump

86 and lines 88 and 80. Scrubber 78 is vented to the atmosphere via line 90.

The nitration reaction in nitration reactor 56 proceeds for a time in the range of 5 to 7 hours after the temperature within reactor 56 has been adjusted to the reflux temperature of the nitric acid. At the termination of the 5 to 7 hour residence time, a portion of the nitric acid reflux can be removed from reflux condenser 70 and introduced via lines 72 and 92 into receiver 94. Approximately 20 to 35 percent by weight of the initial nitric acid charge is removed from the reactor and stored in receiver 94. It has been observed that upon removal of the nitric acid, the temperature within reactor 56 increases to a temperature of about 185 to about 200°F. It has also been observed that completion of the process without removal of nitric acid results in a yield of product of approximately 40 to about 65 weight percent of theoretical yield whereas completion of the process with removal of nitric acid results in a yield of approximately 75 to about 85 weight percent of theoretical. The yield referred to above is predicated upon the quantities of the intermediate BPAP introduced into vessel 56 via lines 62 or 58.

At the end of the 5 to 7 hour retention period in reactor 56 or upon the removal of the portion of nitric acid charge, as described above, the contents of reactor 56 are allowed to cool and upon cooling a slurry of nitric acid and solid product is removed from reactor 56 and introduced into

vacuum separator 96 via line 98. In vacuum separator 96, a liquid filtrate comprised of nitric acid and organic materials is removed via line 100. Upon removal of the filtrate from separator 96, a warm water wash is introduced into vacuum separator 96 via line 102 and liquid is removed via line 100.

Upon completion of washing, the damp solid product, 2,6-bis(picrylamino) - 3,5-dinitropyridine referred to above as PANPX, is removed from filter 96 and introduced via line 104 into dryer 106 wherein it is heated to a temperature of approximately 100°C and maintained in such atmosphere until the quantity of volatile material remaining in the product is less than 0.1 percent by weight. Volatiles are removed from dryer 106 via line 108 and the dried product PANPX is removed from dryer 106 via line 110. As mentioned earlier, the yield of product removed via line 110 is approximately 40 to 65 percent by weight of theoretical when nitric acid is not removed from reactor 56 via lines 92 and 94; however, theoretical yield is in the range of 75 to 85 percent when nitric acid is removed from reactor 56 via lines 72 and 92 and stored in vessel 94.

The following Examples are given to illustrate the invention.

EXAMPLE I

To a vessel equipped with a stirrer was added 100 parts by weight of water. To the water was added 10.9 parts by weight of 2,6-diaminopyridine. At the same time that the water and the 2,6-diaminopyridine were mixing, 400 parts by weight of isopropyl alcohol were introduced into another vessel equipped with a stirrer. 55 parts by weight of picryl chloride were added to the alcohol. After the isopropyl alcohol and picryl chloride were mixed, 8.4 parts by weight of magnesium carbonate were added to the picryl chloride and isopropyl alcohol. Both vessels were stirred and heat was applied to each until the temperature reached 82°F. The 2,6-diaminopyridine was completely dissolved in water and the picryl chloride was dissolved in the isopropyl alcohol. The contents of the vessel containing isopropyl alcohol, picryl chloride, and magnesium carbonate were introduced into a vessel equipped with a stirrer and a reflux condenser. Thereafter, the contents of the vessel containing water and 2,6-diaminopyridine were added to the vessel containing picryl chloride, isopropyl alcohol, and magnesium carbonate. This addition was carried out over a half hour time period and the rate of addition was determined by the rate of evolution of the $CO_2$ by-product. After the addition was completed, the vessel was maintained at the reflux temperature of isopropyl alcohol using external heat. Any vaporized alcohol was condensed and returned to the vessel. All waste

material during the heating such as carbon dioxide was removed using a scrubber containing sodium hydroxide. Upon completion of the reaction, the solid product was filtered and washed with warm water. The intermediate product, 2,6-bis(picrylamino)pyridine (BPAP) was dried at a temperature of about $100^{o}C$ and was maintained at that temperature until the volatiles remaining in the BPAP were less than 0.5 percent by weight. The BPAP had a melting point of $300^{o}C$ and the yield of BPAP was 96 percent of theoretical.

One hundred parts by weight of the BPAP were introduced into a reactor which had been charged with 825 parts by weight of 98 percent nitric acid. The BPAP was introduced at a rate sufficient to maintain a temperature between $32^{o}F$ ($0^{o}C$) and $80^{o}F$ ($30^{o}C$). At certain times cooling of the reactor was required. After the BPAP had been introduced into the reactor, the contents of the reactor were heated slowly to the atmospheric reflux temperature of nitric acid, which is approximately $170^{o}F$ ($76^{o}C$). The contents of the reactor were stirred. All nitric acid vented from the reactor was condensed and returned to the reactor. All noncondensible materials were scrubbed using sodium hydroxide. The reactor was heated for a period of about five hours after the temperature inside the reactor had been heated to the reflux temperature of nitric acid. At the end of five hours, about 20 percent by weight of the nitric acid was removed from the reactor and stored in a receiver. At this time the temperature of the reactor increased to about $200^{o}F$ ($84^{o}C$). After the removal of the desired amount of nitric acid, the contents of the reactor were cooled to about $80^{o}F$ ($27^{o}C$) and introduced into a vacuum separator. A liquid filtrate was removed from the solid material (PANPX) and the solid material was washed. Thereafter, the PANPX was placed into a dryer and heated to a temperature of about $100^{o}F$ ($38^{o}C$). The PANPX was maintained in the dryer until the volatile product was less than 0.1 percent by weight.

The yield of the PANPX was 75 percent of theoretical based on the weight of the intermediate (BPAP) charged. The PANPX was examined by differential scanning calorimetry and found to decompose exothermally at 376°C.

EXAMPLE II

BPAP was prepared in the same manner as Example I except that the BPAP was not dried prior to nitration. The damp BPAP was then introduced into a reactor containing 98 percent nitric acid. The nitration of the BPAP was carried out in the same manner as Example I. The final product (PANPX) was then isolated, washed and dried. The yield of PANPX was found to be 81 percent of theoretical. The PANPX was examined using differential scanning calorimetry and found to decompose exothermally at 372°C.

CLAIMS:

1. A method of preparing 2,6-bis(picrylamino)-3,5 dinitropyridine, comprising:

(a) reacting, in a polar protic solvent, 2,6 diaminopyridine with at least one compound having the general formula

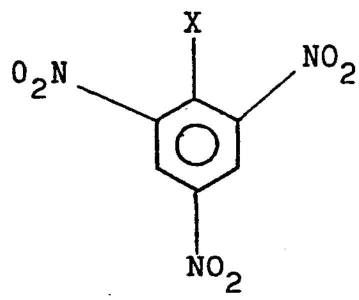

wherein:

X is a halogen or an alkoxy group containing 1 to 4 carbons, provided however that when X is a halogen, at least one base material selected from alkaline earth metal carbonates and alkali metal bicarbonates, is present in an amount of at least one molar equivalent to said compound;

(b) filtering the reaction product of step (a); and

(c) nitrating the filtered reaction product of step (b) with at least 90 percent nitric acid at a temperature in the range of about 32°F (0°C) to about 85°F (30°C).

2. A method according to claim 1, further comprising the step of drying the filtered product of step (b) before nitrating the product in step (c).

3. A method according to claim 1 or 2, wherein in step (c) 98 percent nitric acid is used.

4. A method according to claim 1,2 or 3, wherein in the, or at least one of the, said compound, X is methoxy, ethoxy, isopropoxy or tert-butoxy.

5. A method according to claim 1,2,3 or 4, wherein the (or each) base material is selected from magnesium carbonate, calcium carbonate, barium carbonate, strontium carbonate, lithium carbonate, sodium bicarbonate, potassium bicarbonate, and mixtures thereof.

6. A method according to any of claims 1 to 5, wherein said polar protic solvent is an aqueous alcohol.

7. A method according to claim 6, wherein the alcohol is isopropyl alchohol.

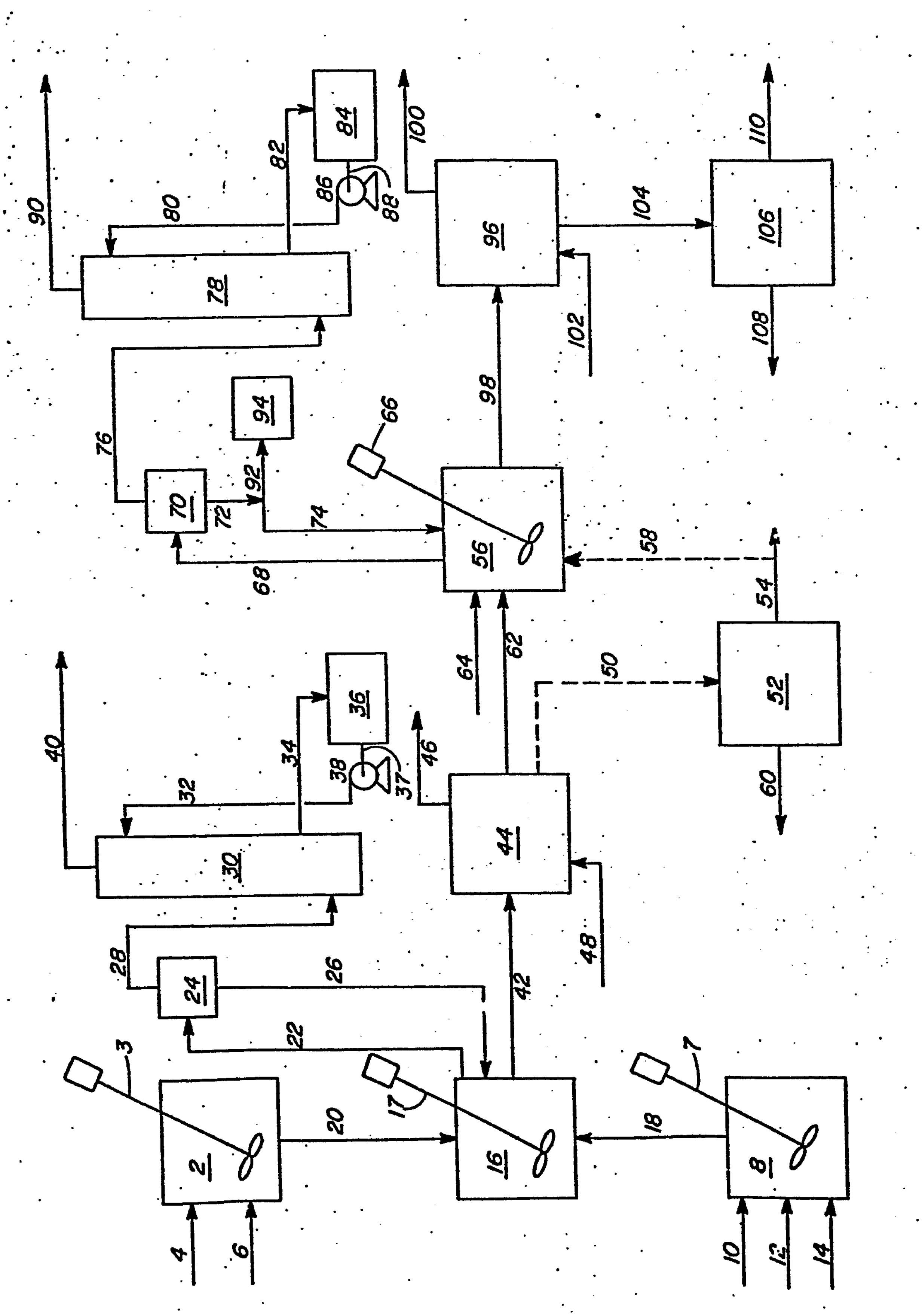
2
3
4
6
7
8
10
12
14
16
17
18
20
22
24
26
28
30
32
34
36
37
38
40
42
44
46
48
50
52
54
56
58
60
62
64
66
68
70
72
74
76
78
80
82
84
86
88
90
92
94
96
98
100
102
104
106
108
110

0104717

European Patent Office

# EUROPEAN SEARCH REPORT

Application number

EP 83 30 3684

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| D,A | US-A-3 678 061 (UNITED STATES ATOMIC ENERGY COMMISSION) * Complete document * --- | 1 | C 07 D 213/74 |
| A | Chemical Abstracts vol. 93, no. 18, 3 November 1980, Columbus, Ohio, USA J.C. ADENIS et al. "Syntheses and properties of some thermally stable ignition explosives", page 136, right-hand column, abstract no. 170504e & Eur. Space Agency, ESA SP-144, 1980, pages 69-76 --- | 1 | |
| A | Chemical Abstracts vol. 78, no. 3, 22 January 1973, Columbus, Ohio, USA M.D. COBURN et al. "Picrylamino-substituted heterocycles. V. Pyridines", page 377, column 1, abstract no. 15984h J. Heterocycl. Chem., vol. 9, no. 5, 1972, pages 1 039-1044 --- | 1 | TECHNICAL FIELDS SEARCHED (Int. Cl. ³) C 07 D 213/74 |
| A | Soviet Inventions Illustrated Week C 08, 2 April 1980 Section E 132 & SU-A-667552 ----- | 1 | |

The present search report has been drawn up for all claims

| Place of search | Date of completion of the search | Examiner |
|---|---|---|
| BERLIN | 17-11-1983 | PHILLIPS N.G.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document